# EUROPEAN PATENT APPLICATION

(11) **EP 4 427 711 A1**
(43) Date of publication of application: **11.09.2024**
(21) Application number: 22885842.9
(22) Date of filing: 24.10.2022
(51) Int. Cl.: A61F 2/82, A61F 2/89, A61F 2/07

(54) **LUMINAL STENT**

(30) Priority: 01.11.2021 CN 202111284850
(71) Applicant: Lifetech Scientific (Shenzhen) Co., Ltd., Shenzhen, Guangdong 518063 (CN)
(72) Inventor: XIAO, Benhao, Shenzhen, Guangdong 518063 (CN); TANG, Chunwei, Shenzhen, Guangdong 518063 (CN)
(74) Representative: Prinz & Partner mbB
(86) International application number: PCT/CN2022/126922
(87) International publication number: WO 2023/071965

(57) **Abstract**

A lumen stent (100) includes a tubular stent and an imaging marker; the tubular stent includes a main body stent (11); the imaging marker includes a first marker (211) provided on the main body stent (11); and the shape of the first marker (211) provided on the main body stent (11) is asymmetrical at least along a longitudinal axis. A non-axisymmetric imaging marker is provided on the main body stent (11), and the position and orientation of the lumen stent (100) implanted in the body can be accurately and clearly displayed through the imaging marker, to allow the operator to observe and quickly judge and timely adjust, thereby shortening the operation time of the endovascular treatment and reducing the difficulty of the operation.

## Description

### Technical Field

The present invention relates to the technical field of interventional medical devices, and in particular to a lumen stent.

### Background Art

Endovascular tubular stent exclusion of the aorta has been widely used in thoracic and abdominal aortic aneurysms and arterial dissection for more than ten years. It has been a first-line treatment with definite efficacy, less trauma, rapid recovery, and fewer complications. During the operation, under the supervision of X-ray fluoroscopy, the tubular stent is sent to the lesion location through the corresponding delivery system. The tubular stent will isolate the blood flow from the lesion location and eliminate the influence of blood pressure on the lesion location, to achieve the purpose of cure.

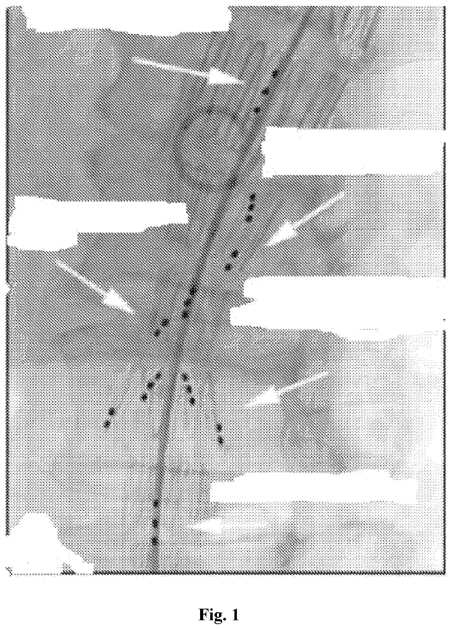

How to accurately position the stent in the body is one of the key conditions for successful endovascular treatment. In common lumen stents, the imaging material is set at the stent covering film port as the positioning indicator, while in the endovascular treatment of arterial diseases with lesions involving branch vessels, the lumen stent is generally designed with branches/windows. For the lumen stent with branches/windows, how to quickly and accurately position the stent in the lumen is more important. Existing positioning methods of the stent with branches/windows, such as COOK's t-Branch stent's imaging markers, as shown in Fig. 1, use ".-shaped" imaging markers to position the proximal end and distal end of the covering film, and four outer branch ports. The problems with such imaging markers are that the number of markers is large and the markers with different characteristics are not easily distinguished, intraoperative observation is difficult to judge, and since the markers are visualized under DSA digital imaging equipment, therefore, the ".-shaped" design of the COOK Company stent will present the same ".-shaped" imaging shape in the imaging process regardless of whether the stent is placed on the front side or the reverse side, which means that the operator often cannot distinguish the anteroposterior orientation of the circumferential direction of the stent. If the improper anteroposterior orientation is found after the release of the lumen stent during the surgery, the endovascular treatment surgery will fail. The lumen stent placed in the wrong position will affect the normal blood supply of the patient's blood vessels. The stent can only be removed by emergency surgery, and the patient is then exposed to serious life risks.

### Summary of the Invention

The technical problem to be solved by the present invention is to provide a lumen stent given the above-mentioned defects in the prior art.

The technical solution used by the present invention to solve the technical problem is:
Provided is a lumen stent including a tubular stent and an imaging marker; the tubular stent includes a main body stent; the imaging marker includes a first marker provided on the main body stent; and the shape of the first marker provided on the main body stent is asymmetrical at least along a longitudinal axis.

In one embodiment, the main body stent includes an opposite front half and back half; the first marker is provided on the front half or the back half; the shape of an orthographic projection of the first marker on a plane intersecting the front half and the back half is approximately the same as its shape.

In one embodiment, the shape of the first marker is non-axisymmetric.

In one embodiment, the tubular stent further includes a branch stent, the branch stent has an opposite distal opening and proximal opening, and one of the distal openings and the proximal openings is connected to a sidewall of the main body stent such that a lumen of the branch stent communicates with a lumen of the main body stent; the first marker is located on the main body stent at an end of the distal opening of the branch stent.

In one embodiment, the imaging marker further includes an annular marker provided at an edge of or near the distal opening and/or the proximal opening of the branch stent.

In one embodiment, the distal opening and/or the proximal opening of the branch stent are arranged obliquely with respect to a longitudinal central axis of the branch stent such that the annular marker is also oblique with respect to the longitudinal central axis of the branch stent.

In one embodiment, the main body stent includes an opposite main proximal opening and main distal opening; the imaging marker further includes a second marker provided adjacent to the main proximal opening.

In one embodiment, the imaging marker further includes a third marker provided adjacent to the main proximal opening; the second marker and the third marker have different shapes; the second marker and the third marker are spaced apart circumferentially along the main body stent, and the radial line of the second marker and the third marker passes through the longitudinal central axis of the main body stent.

In one embodiment, the radial line of the first marker and the longitudinal central axis of the main body stent are perpendicular to the radial line of the second marker and the third marker.

In one embodiment, the main body stent further includes a fourth marker provided adjacent to the main distal opening.

In one embodiment, the fourth marker is on the same longitudinal line as the first marker.

In one embodiment, the imaging marker further includes a fifth marker provided on the main body stent at a preset spacing from the main distal opening, the preset spacing being no less than 20 mm.

In one embodiment, the fifth marker is on the same longitudinal line as the first marker.

In summary, the present invention provides a non-axisymmetric imaging marker on the main body stent, and the position and orientation of the lumen stent implanted in the main body can be accurately and clearly displayed through the imaging marker, to allow the operator to observe and quickly judge and timely adjust positioning, thereby shortening the procedure time of the endovascular treatment and reducing the difficulty of the operation.

### Brief Description of the Drawings

The present invention will now be further described, by way of example, concerning the accompanying drawings, in which:
Fig. 1 is the imaging shape of an imaging marker of a conventional lumen stent under X-ray;
Fig. 2 is a front view of an exemplary lumen stent according to the present invention;
Fig. 3 is a rear view of an exemplary lumen stent according to the present invention;
Fig. 4 is the shape of a first symbolic marker after left torsion of a lumen stent;
Fig. 5 is the shape of a first symbolic marker after right torsion of a lumen stent; and
Fig. 6 is the imaging shape of an annular marker on a branch stent of an exemplary lumen stent under X-ray according to the present invention.

### Detailed Description of the Invention

To make the above objects, features, and advantages of the present invention more apparent, a detailed description of the embodiments of the present invention will be given below concerning the accompanying drawings. In the following description, numerous specific details are outlined to provide a thorough understanding of the present invention. The present invention may, however, be embodied in many different forms than described herein, and modifications may be made by those skilled in the art without departing from the spirit of the present invention so that the present invention is not limited to the specific embodiments disclosed below.

It will be understood that when an element is referred to as being "fixed" or "provided" on another element, it can be directly on the other element or intervening elements may also be present. When an element is referred to as being "connected" to another element, it can be directly connected to the other element or intervening elements may also be present. As used herein, the terms "vertical", "horizontal", "left", "right", and the like are used for descriptive purposes only and are not intended to be exclusive implementations.

Unless defined otherwise, all technical and scientific terms used herein have the same meaning as commonly understood by the ordinarily skilled in the art to which the present invention belongs. The terminology used herein in the description of the present invention is to describe specific implementations only and is not intended to be limited to the present invention. As used herein, the term "and/or" includes any combinations of one or more of the associated listed items.

In the field of interventional medicine, an implant (such as a lumen stent) is generally defined as proximal at the end that is proximal to the heart and distal at the end that is distal to the heart after release.

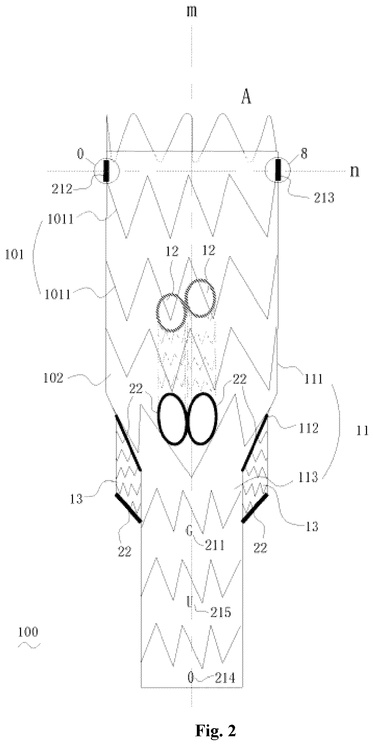

Referring to Fig. 2, one embodiment of the present application provides a lumen stent 100 including a tubular stent and an imaging marker provided on the tubular stent. The tubular stent includes a main body stent 11, and the imaging marker includes a first marker 211 provided on a peripheral wall of the main body stent 11; the shape of the first marker 211 provided on the main body stent 11 is asymmetric at least along a longitudinal axis. The longitudinal axis refers to a longitudinal line passing through the first marker 211, the longitudinal line dividing the first marker 211 into two halves on the left and right sides of the longitudinal line, and the left and right halves of the first marker 211 are asymmetric along the longitudinal line. When the first marker 211 of the embodiment is asymmetric along the longitudinal axis, when the X-ray fluoroscopy is a planar image, and the orientation of the lumen stent is different, the lumen stent presents different imaging shapes, which can not only indicate the orientation in the axial direction but also can be quickly identified in the circumferential direction, thereby allowing the operator to adjust the stent release orientation in time, greatly reducing the operation time and improving the operation success rate.

Preferably, in some embodiments, the shape of the first marker 211 is non-axisymmetric, that is, the first marker 211 is a non-axisymmetric marker that does not itself have any axis of symmetry. In the embodiment, a non-axisymmetric first marker 211 is used, and the first marker 211 is pre-fixed on the tube of the main body stent 11. When on the wall, making the appearance of the first marker 211 to the observer's fixed visual field unique, and the position relationship and orientation relationship of the unique shape concerning the main body stent 11 and the position relationship and orientation relationship concerning each branch/window on the main body stent 11 are unique and fixed, and therefore, taking this as a reference, during the implantation of the lumen stent, observing the imaging shape shown by the first marker 211, and then adjusting the position of the lumen stent according to the known relative position relationship and orientation relationship, so that the imaging shape presented is the same as the pre-set shape. Thus, the lumen stent is accurately and quickly implanted into the preset position. Unlike the ".-shaped " marker of the prior art, the non-axisymmetric first marker 211 of the embodiment establishes a unique position and orientation relationship with the stent via the symbol shape, and the imaging marker can accurately display the position and orientation of the stent implanted in the main body, to allow the operator to observe and quickly judge and timely adjust, thereby shortening the operation time of the endovascular treatment and reducing the difficulty of the operation.

Further, the first marker 211 of this embodiment is a character-shaped imaging marker to facilitate production and provide a clear imaging marker. Preferably, as shown in Fig. 2, the symbol shape of the first marker 211 is G-shaped. For the present application, the G-shape is not only relatively uniform in shape and has strong instability, but more importantly, has a high degree of discrimination after imaging. Referring to Fig. 2, a detailed description is given of the process of imaging the identification of the G-shaped first marker 211, the G-shaped first marker 211 is fixed in the middle of the lumen stent 100, so that the distance from the proximal end port of the lumen stent 100 and the distance from the distal end port is fixed, so that the axial position of the lumen stent can be identified by the distance during the implantation. Furthermore, since the G-shaped first marker 211 is located directly in front of the front half A of the stent when the lumen stent is normally implanted, the imaging shape of the first marker 211 when the X-ray fluoroscopy is a planar image should be approximately the same as its symbol shape, so that the imaging shape seen by the operator is G-shaped under normal conditions. However, if the visual appearance is non-G-shaped, the lumen stent is distorted during implantation; if the visual appearance is an inverted G-shape, it means that the lumen stent is twisted 180 °, namely, the orientation of the front half A and back half B of the lumen stent is reversed; if a significant narrowing of the width of the imaging shape is seen, a left or right twist occurs.

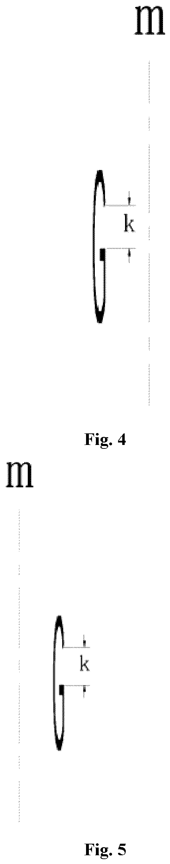

As shown in Fig. 4, if the line with the discontinuity k on the right side of the imaging shape with a significantly narrowed width is closer to the central axis m, a left torsion is demonstrated and the lumen stent needs to be rotated to the right. As shown in Fig. 5, if the continuous line on the left side of the imaging shape having a significantly narrowed width is closer to the central axis m, it is confirmed that a right torsion occurred, and the lumen stent needs to be rotated to the left to identify the circumferential position of the lumen stent. Thus, the lumen stent can be quickly and accurately positioned by the above-described non-axisymmetric G-shaped first marker 211 having a high degree of discrimination. It should be noted that the symbol shape of the first marker 211 is not limited to a G-shape, and symbols of a symbol shape having a high degree of recognition, which is non-axisymmetric, are within an optional range.

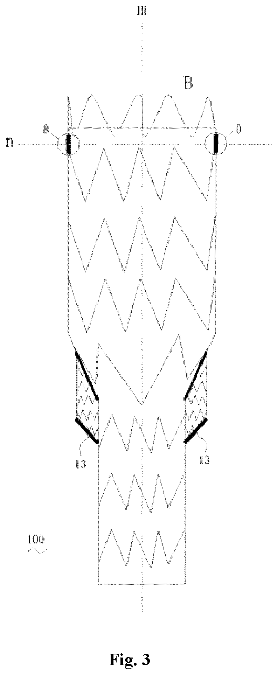

For one embodiment, as shown in connection with Figs. 2 and 3, the main body stent 11 includes two opposite halves (A and B), respectively a front half A and a back half B, a first marker 211 is provided on the front half A or the back half B, and the shape of the orthographic projection of the first marker 211 on a plane intersecting the front half A and the back half B is approximately the same as the shape thereof. Preferably, to facilitate the observation and to conform to the observation habit of the operator, as shown in Fig. 2, the first marker 211 is provided on the front half A, and the G-shaped first marker 211 in Fig. 2 has a G-shaped orthographic projection on the plane. Generally, during the implantation of the lumen stent, the front half A of the main body stent 11 is located above (i.e. directly in front of the upper operator of the patient) so that when the first marker 211 is provided on the front half A, on the one hand, it can present a clear imaging shape for easy observation; on the other hand, the imaging shape of the first marker 211 is consistent with the self-symbol shape of the first marker 211 observed under a normal viewing angle, so that there is no problem with mirror observation, etc. which is more consistent with the usage habit of the operator. Of course, the first marker 211 may also be provided on the back half B, which may be provided according to actual needs. When the lumen stent is normally implanted, it can be further defined that the first marker 211 has approximately the same imaging shape as its shape when the X-ray fluoroscopy is a planar image. That is, when the lumen stent is normally implanted, the shape of the orthographic projection of the first marker 211 is approximately the same as its shape. In other words, when the patient lies flat for normal implantation of the lumen stent, the first marker 211 is located directly above or directly below the wall of the lumen stent, and at this time, when the X-ray fluoroscopy is a planar image, the imaging shape is the shape of the orthographic projection of the first marker 211 located directly above or directly below, and this shape is approximately the same as its shape. It should be noted that, since it is judged whether the same or not is judged by the naked eye of the operator during the actual operation, the embodiment is limited to be approximately the same, allowing a certain error range, which should not affect the normal implantation of the lumen stent.

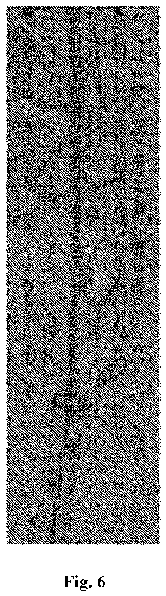

Further, in some embodiments, the tubular stent includes a branch stent having opposite distal openings and proximal openings, one of the distal openings and proximal openings being connected to the sidewall of the main body stent such that the lumen of the branch stent is in communication with the lumen of the main body stent; the first marker 211 is located on the peripheral wall of the main body stent 11 at the end of the distal opening of the branch stent. In conjunction with the foregoing description, the first marker 211, which is asymmetric along the longitudinal axis at this time, presents different imaging shapes when the X-ray fluoroscopy is a planar image, and at the same time, the first marker 211 is located at the distal end of the distal opening of the branch stent; the first marker 211 can not only facilitate the accurate positioning of the lumen stent in the axial and circumferential directions but also can identify the axial position of the stent's half-release. As shown in connection with Figs. 2 and 6, the release of the stent system sheath orifice is paused at the first marker 211 of the G-shape, at which time the branch stent is just fully released, the first marker 211 further enables the positioning of the lumen stent half-release process. The first marker 211 of the embodiment not only facilitates the "all-purpose" but also has a characteristic shape to provide a clear and accurate positioning function.

Illustratively, with continued reference to Fig. 2, the tubular stent includes two inner branches 12 and two outer branches 13 in communication with the main body stent 11. The main body stent 11, the inner branch 12, and the outer branch 13 are all of a tubular stent structure having a hollow lumen, the hollow lumen constituting a passage for blood flow. It should be noted that the placement position, the number of the branch stents, and the length of the branch stents are not limited thereto, and may be set according to actual needs.

As shown in Fig. 2, the main body stent 11, the inner branch 12, and the outer branch 13 each include a bare stent 101, and a covering film 102 connected to the bare stent 101. The bare stent 101 is made of materials having good biocompatibility, such as nickel-titanium and stainless steel. The covering film 102 is made of polymer materials with good biocompatibility, such as PTFE, FEP, and PET. Bare stent 101 includes a plurality of waveform rings 1011; the plurality of waveform rings 1011 are arranged in sequence from proximal end to distal end, preferably in parallel spaced arrangement. The waveform ring 1011 has a closed cylindrical structure, and the waveform ring 1011 may have the same or similar waveform shape among the rings. For example, the waveform ring 1011 may have a Z-shaped wave, an M-shaped wave, a V-shaped wave, a sine-shaped wave structure, or other structures radially compressible to a very small diameter, etc. It is to be understood that the embodiment is not limited to the specific configuration of the waveform ring 1011, and the waveform shape of the waveform ring 1011 may be set as required, and the number of waveform shapes and the height of the waveform shape in each turn of the waveform ring 1011 may be set as required. In actual preparation, the bare stent 101 may be cut and shaped using a nickel-titanium tube, and then the bare stent 101 may be sutured onto the covering film 102. As an embodiment, concerning Fig. 2, the main body stent 11 includes a first main body segment 111, a tapered segment 112, and a second main body segment 113 connected in sequence, the cross-sectional area of the first main body segment 111 is greater than the cross-sectional area of the second main body segment 113, and the two inner branches 12 and the two outer branches 13 are both connected to the tapered segment 112. The distal end of the inner branch 12 is fixed on the tapered segment 112, and the proximal end is located inside the main body stent 11 and extends towards a side away from the second main body segment 113; the proximal end of the outer branch 13 is secured to the tapered segment 112 and the distal end is external to the main body stent 11 and extends to a side remote from the first main body segment 111.

During surgery, the first main body segment 111 is first attached to the healthy vessel wall upstream of the tumor cavity, and the tapered segment 112 and the second main body segment 113 remain in the tumor cavity. A guide wire (not shown) is then passed from within the inner branch 12 or the outer branch 13 and introduced into the branch vessel near the tumor lumen to establish a trajectory. Then, one end of the branch stent is telescoped into the inner branch 12 or the outer branch 13, and the other end of the branch stent is positioned in the branch vessel through which the blood flow passing from the main body stent 11 is introduced.

In the present application, two inner branches 12 and two outer branches 13 are connected to a tapered segment 112, and since the tapered segment 112 is closer to the first main body segment 111 than the second main body segment 113, the guide wire has more operation space after passing out from the distal end of the inner branch 12 or the outer branch 13, to facilitate accurate introduction of the guide wire into a branch vessel. Furthermore, by providing two inner branches 12 and two outer branches 13 on the tapered segment 112, the distal ends of the inner branches 12 and the outer branches 13 can be positioned in different planes, thereby enabling the offset placement of the branch stents within the tumor, and avoiding pinching between the branch stents.

Furthermore, since the X-ray fluoroscopic image at the time of surgery is a planar image, if the two outer branches 13 are spaced too close to each other, the imaging markers of the two outer branches will interfere, which is not conducive to the selection of the guide wire into the corresponding branch, affecting the surgical operation and extending the surgical time, so the farther the distance between the two outer branches is, the better. By the same reasoning, if the adjacent inner branch 12 is spaced too close to the outer branch 13, it will lead to connecting the sleeved branch stent on the inner branch first and then affecting the guide wire to be selected into the outer branch 13, and the imaging markers of the inner and outer branches will also interfere, so the farther the distance between the inner and outer branches is, the better. If the two inner branches and the two outer branches are symmetrically spaced apart, the imaging markers of the two inner branches can easily interfere under the plane perspective image, so that the two inner branches 12 are located between the two outer branches 13. Therefore, it is necessary to rationally design the position between the inner branch 12 and the outer branch 13 to avoid the interference of the imaging markers between the four branches and to avoid having the intervals between the branches to be too close to affect the guide wire selection. In the embodiment, the two inner branches 12 are located between the two outer branches 13.

With continued reference to Fig. 2, the main body stent 11 is provided with four windows, two of which are inner branch windows connected to the inner branch 12 and two of which are outer branch windows connected to the outer branch 13. In other embodiments, the imaging marker further includes an annular marker 22 along or near the edge of the distal opening and/or proximal opening of the branch stent. That is, annular markers 22 are provided at or near the edges of the four windows, and annular markers 22 are also provided at or near the edges of the free ports of the two inner branches 12 and the two outer branches 13. The annular marker 22 can visualize the exit and entrance of the branch stent to facilitate the penetration and exit of the guidewire. Whether or not annular markers 22 are provided at or near the edge of the end opening of the branch stent may be provided as desired, without limiting the exemplary illustration described above.

Furthermore, in actual surgery, how to quickly identify the outlet and inlet of the branch stent to enable the guide wire to quickly extend into and out is particularly important, but the fact is that the X-ray fluoroscopy image at present surgery is planar, while the planar image presented by the outlet and inlet of the common branch stent with a horizontal section port is a horizontal straight short line, which results in the operator being unable to identify the outlet and inlet of the branch stent, and also unable to know the deployment state of the branch stent. Given this, concerning Figs. 2 and 6, the distal opening and/or proximal openings of the branch stent of the embodiment are provided obliquely with respect to the longitudinal central axis of the branch stent such that the annular marker 22 is also oblique with respect to the longitudinal central axis of the branch stent. Typically, the angle of inclination is greater than 10 degrees. The obliquely arranged port and the annular marker 22 in the embodiment allow the entrance and exit of the branch stent to present a three-dimensional shape after being imaged, and the annular structure is more convenient for the operator to identify the deployment shape of the branch entrance and exit, and can also further determine whether the branch stent is fully deployed according to the deployment shape of the entrance and exit, facilitating the establishment of the channel of the branch stent. In addition, the obliquely provided annular marker 22 is easily distinguishable from the main body imaging marker and is more easily radially compressed with a smaller cross-section so that the profile of the stent compressed in the sheath can be reduced, facilitating a reduction in the sheath diameter.

Referring to Fig. 2, to position the axial proximal end of the lumen stent, the main body stent 11 includes opposite main proximal opening and main distal openings, and the imaging marker further includes a second marker 212 provided adjacent to the main proximal opening. The circumferential orientation of the second marker 212 may be fixed as desired. To improve the accuracy of the axial positioning, the second marker 212 is placed as close to the port as possible.

Furthermore, since the proximal port of the lumen stent is the primary positioning position at the time of implantation of the lumen stent, the accuracy of the proximal port positioning is particularly important. In other embodiments, to improve the accuracy of the circumferential positioning at the same time as the accurate axial positioning, the imaging marker further includes a third marker 213 arranged adjacent to the main proximal opening; the second marker 212 and the third marker 213 have different symbolic shapes, and the second marker 212 and the third marker 213 are arranged at intervals along the circumferential direction of the main body stent. The location can be made by two markers with different symbols of orientation. Preferably, to further enhance the identification of degrees during circumferential positioning, a radial line n connecting the second marker 212 and the third marker 213 passes through the central axis m of the main body stent 11. That is, the radial line n of the second marker 212 and the third marker 213 is located on the half-cut plane of the front and rear portion of the main body stent 11, i.e. as shown in Fig. 2, the second marker 212 and the third marker 213 are located on the left and right sides of the lumen stent, respectively. At this time, a radial line connecting the first marker 211 with the central axis m of the main body stent 11 is perpendicular to the half-cut plane. Namely, the second marker 212, the third marker 213, and the first marker 211 are T-shaped in space. In this way, during normal implantation, the second marker 212 and the third marker 213 present two vertical short lines of imaging shape, and if torsion occurs, the imaging shape of the second marker 212 and the third marker 213 will change, so it is possible to further judge whether the orientation of the lumen stent is accurate based on the first marker 211, and improve the identification and accuracy.

Referring to Fig. 2, for more precise positioning of the axial distal end of the lumen stent, the main body stent 11 further includes a fourth marker 214 provided adjacent the main distal opening. The orientation of the fourth marker 214 in the circumferential direction may be fixed as required. To improve the accuracy of the axial positioning, the fourth marker 214 is placed as far as possible from the port. Preferably, the first marker 211 and the fourth marker 214 are located on the same longitudinal line, and a radial line connecting the first marker 211 and the fourth marker 214 to the central axis m of the main body stent 11 is perpendicular to a radial line connecting the second marker 212 and the third marker 213. In this way, it is also ensured that the symbolic shape of the fourth marker 214 is visible during normal implantation.

Illustratively, as shown in Fig. 2, the second marker 212, and/or the third marker 213, and/or the fourth marker 214 are character-shaped imaging markers having a symbol shape of 0 or 8. The choice of 0-shape or 8-shape provides, on the one hand, a symbol that has a smooth closure as a whole, without any sharp spikes, and without any influence on the vessel wall during the contact of the proximal end of the lumen stent with the vessel wall. On the other hand, the symbol shape is uniform and the fixed stability is good.

It will be appreciated that since the symbol shapes of the second marker 212 and the third marker 213 are different for ease of recognition, the symbol shape of the third marker 213 may be selected to be 8-shaped when the symbol shape of the second marker 212 is 0-shaped. To facilitate suture fixation, the fourth marker 214 has a 0-shaped symbol shape. It should be noted that the selection of the symbols of the second marker 212, the third marker 213, and the fourth marker 214 is not limited thereto, and the selection setting may be continued according to actual needs.

Referring to Fig. 2, if the distal end of the lumen stent needs to be connected to the extension stent, the imaging marker further includes a fifth marker 215 provided on the main body stent at a preset spacing from the main distal opening; the preset spacing is not less than 20 mm. The proximal end of the covering film of the extended stent can be axially positioned by the fifth marker 215, and the preset spacing of not less than 20 mm effectively ensures the necessary length of an overlapping segment, to avoid the detachment of the extended stent. Preferably, the fifth marker has the shape of a U. Preferably, the first marker 211 and the fifth marker 215 are located on the same longitudinal line, and a radial line connecting the first marker 211 and the fifth marker 215 to the central axis m of the main body stent 11 is perpendicular to a radial line connecting the second marker 212 and the third marker 213. In this way, it is also ensured that the symbolic shape of the fifth marker 215 is visible during normal implantation.

Further, when the first marker 211, the fourth marker 214, and the fifth marker 215 are provided at the same time, the first marker 211, the fourth marker 214 and the fifth marker 215 are located on the same line, and a radial line connecting the first marker 211, the fourth marker 214 and the fifth marker 215 to the central axis m of the main body stent 11 is perpendicular to the radial line connecting the second marker 212 and the third marker 213. That is, the second marker 212, the third marker 213, the first marker 211, the fourth marker 214, and the fifth marker 215 are in a T-shape arrangement in a linear space. In this way, during normal implantation, the second marker 212 and the third marker 213 present two vertical short lines in the imaging shape, while the first marker 211, the fourth marker 214, and the fifth marker 215 present a complete and clear symbol shape, positioning the lumen stent in all directions and at various angles, thereby facilitating rapid and accurate positioning. Concerning Fig. 2, the embodiment provides two imaging markers (respectively a second marker 212 and a third marker 213) with different symbols for precisely positioning the proximal position on both sides of the proximal port, a fourth marker 214 for precisely positioning the distal position is provided directly in front of the distal port, an asymmetric first marker 211 for achieving axial and circumferential and complete release is provided directly in front of the intermediate position, and a fifth marker 215 for axially positioning the proximal end of the extension stent covering film is provided between the first marker 211 and the fourth marker 214. The shape function of each marker is not repeated, so it can be clearly and quickly identified to the operator without confusion.

The first marker 211, the second marker 212, the third marker 213, the fourth marker 214, and the fifth marker 215 in the embodiment are rigid imaging markers. On the one hand, the volume of the rigid imaging marker is small, which will not significantly increase the radial dimension of the stent after extrusion, so as to make it easy to sheath; on the other hand, the shape of the rigid 5-D marker does not change due to the loading of the lumen stent into the sheath, and the shape of the marker before and after release is the same. In this way, the operator can see the marker on the lumen stent in the sheath through the DSA digital imaging equipment before releasing the lumen stent, so that the orientation of the sheath can be adjusted in time to change the orientation of the lumen stent before releasing, to further ensure the accurate positioning of the lumen stent during releasing. The annular marker 22 of the embodiment is a flexible imaging marker. The flexible annular marker 22, on the one hand, is easily squeezed and does not cause an increase in the radial dimension of the lumen stent after squeezing, facilitating sheathing. On the other hand, the flexible annular marker 22 is easily deployed after release, and it can be further determined whether the branch stent is well deployed by the deployed shape of the annular marker 22.

Further, the outer wall of the first main body segment 111 is provided with a barbed structure (not shown) to enhance the anchoring performance of the tubular stent as a whole. When other stents are overlaid at the proximal end of the first main body segment 111 if the barbed structure is too close to the proximal end of the first main body segment 111, the barbed structure may easily puncture the other stents and form endoleak, but if the barbed structure is too close to the inner branch 12, the flexibility of the lumen stent as a whole may be compromised. Thus, the barbed structure is secured to the outer wall of the first main body segment 111 between the proximal end of the inner branch 12 and the proximal end of the first main body segment 111.

Further, the outer surface of the lumen stent 100 is provided with a semi-releasing means (not shown) to accurately position the lumen stent 100.

Since the lumen stent 100 is mainly axially and circumferentially positioned using the imaging markers thereon when the lumen stent 100 is compressed in the delivery sheath, it has compression wrinkles in the circumferential direction and is axially elongated, and if it is positioned through the imaging markers at this time, there will be a large circumferential and axial deviation. In the present application, a semi-release device is provided on the outer surface of the lumen stent 100; when the lumen stent 100 is completely released from the delivery sheath, the lumen stent 100 is in a semi-release state under the constraint of the semi-release device, and at this time, the lumen stent 100 does not fit with the vessel wall; the operator can still adjust the axial and circumferential positions of the lumen stent 100; and after accurate positioning, the constraint of the semi-release device is released, so that the lumen stent 100 is unfolded and opposed. It can be understood that if the diameter of the circumscribed circle of the cross-section of the lumen stent 100 is too large when the stent is in the semi-released state, the stent is easy to adhere to the wall, which is not conducive to the axial and circumferential adjustment; if the diameter of the circumscribed circle of the cross-section of the stent 100 is too small when the stent is in the semi-released state, the role of the semi-release is not great, and there is still a large circumferential and axial positioning deviation. Therefore, in the embodiment, the ratio of the diameter of the circumscribed circle of the cross-section of the lumen stent 100 in the semi-released state to the diameter of the circumscribed circle of the cross-section of the lumen stent 100 in the deployed state is 0.6 to 0.8.

In the present invention, a non-axisymmetric imaging marker is provided on the main body stent, and the position and orientation of the lumen stent implanted in the main body can be displayed accurately and clearly through the imaging marker, to facilitate the operator to observe and quickly judge and timely adjust, thereby shortening the operation time of the endovascular treatment and reducing the difficulty of the operation.

Each technical feature of the above-mentioned embodiments can be combined in any combination, and to make the description concise, not all the possible combinations of each technical feature in the above-mentioned embodiments are described; however, as long as there is no contradiction between the combinations of these technical features, they should be considered as the scope of the description.

The embodiments described above represent only a few embodiments of the present invention and are described in more detail and are not to be construed as limiting the scope of the present invention. It should be noted that several variations and modifications can be made by one skilled in the art without departing from the inventive concept, which is within the scope of the present invention. Accordingly, the protection sought herein is as outlined in the claims below.

## Claims

1. A lumen stent, comprising a tubular stent and an imaging marker, wherein the tubular stent comprises a main body stent; the imaging marker comprises a first marker provided on the main body stent; and the shape of the first marker provided on the main body stent is asymmetrical at least along a longitudinal axis.

2. The lumen stent according to claim 1, wherein the main body stent comprises an opposite front half and back half; the first marker is provided on the front half or the back half; the shape of an orthographic projection of the first marker on a plane intersecting the front half and the back half is approximately the same as its shape.

3. The lumen stent according to claim 1, wherein the shape of the first marker is non-axisymmetric.

4. The lumen stent according to claim 1, wherein the tubular stent further comprises a branch stent, the branch stent has an opposite distal opening and proximal opening, and one of the distal opening and the proximal opening is connected to a sidewall of the main body stent such that a lumen of the branch stent communicates with a lumen of the main body stent; the first marker is located on the main body stent at an end of the distal opening of the branch stent.

5. The lumen stent according to claim 4, wherein the imaging marker further comprises an annular marker provided at an edge of or near the distal opening and/or the proximal opening of the branch stent.

6. The lumen stent according to claim 5, wherein the distal opening and/or the proximal opening of the branch stent are arranged obliquely with respect to a longitudinal central axis of the branch stent such that the annular marker is also oblique with respect to the longitudinal central axis of the branch stent.

7. The lumen stent according to any one of claims 1 to 6, wherein the main body stent comprises an opposite main proximal opening and main distal opening; the imaging marker further comprises a second marker provided adjacent to the main proximal opening.

8. The lumen stent according to claim 7, wherein the imaging marker further comprises a third marker provided adjacent to the main proximal opening; the second marker and the third marker have different shapes; the second marker and the third marker are spaced apart circumferentially along the main body stent, and the radial line of the second marker and the third marker passes through a longitudinal central axis of the main body stent.

9. The lumen stent according to claim 8, wherein the radial line of the first marker and the longitudinal central axis of the main body stent is perpendicular to the radial line of the second marker and the third marker.

10. The lumen stent according to claim 7, wherein the main body stent further comprises a fourth marker provided adjacent to the main distal opening.

11. The lumen stent according to claim 10, wherein the fourth marker is on the same longitudinal line as the first marker.

12. The lumen stent according to claim 7, wherein the imaging marker further comprises a fifth marker provided on the main body stent at a preset spacing from the main distal opening, the preset spacing being no less than 20 mm.

13. The lumen stent according to claim 12, wherein the fifth marker is on the same longitudinal line as the first marker.
